# EUROPEAN PATENT SPECIFICATION

(11) **EP 1 360 176 B1**
(45) Date of publication and mention of the grant of the patent: **02.11.2006**
(21) Application number: 02719787.0
(22) Date of filing: 08.02.2002
(51) Int. Cl.: C07D 207/34, A01N 43/36

(54) **PYRROLECARBOXAMIDES FOR USE AS FUNGICIDES**
PYRROLKARBOXAMID-DERIVATE UND DEREN EINSATZ ALS FUNGIZIDE
PYRROLECARBOXAMIDES UTILISES EN TANT QUE FONGICIDES

(30) Priority: 09.02.2001 GB 0103258
(43) Date of publication of application: 12.11.2003
(73) Proprietor: Syngenta Participations AG, 4058 Basel (CH)
(72) Inventor: WALTER, Harald, Syngenta Crop Protection AG, CH-4058 Basel (CH)
(74) Representative: Hölscher, Ingo
(86) International application number: PCT/EP2002/001344
(87) International publication number: WO 2002/064562

(56) References cited:
- EP-A- 0 219 756
- WO-A-00/09482
- WO-A-97/08148

## Description

The present invention relates to novel pyrrolecarboxamides which have microbiocidal activity, in particular fungicidal activity. The invention also relates to the preparation of these substances, to agrochemical compositions which comprise at least one of the novel compounds as active ingredient, to the preparation of the compositions mentioned and to the use of the active ingredients or compositions in agriculture and horticulture for controlling or preventing infestation of plants by phytopathogenic microorganisms, preferably fungi.

The pyrrolecarboxamides of the present invention have the general formula I 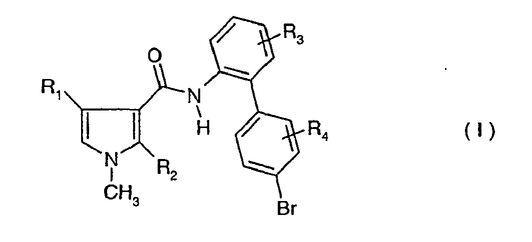 wherein
R₁ is CF₃, CF₂H or CFH₂;
R₂ is hydrogen or fluoro;
R₃ is hydrogen or fluoro; and
R₄ is hydrogen, fluoro, chloro, bromo, methyl, CF₃, OCF₃ or SCF₃.

Surprisingly, it has now been found that the compounds of formula I exhibit improved biological properties which render them more suitable for the practical use in agriculture and horticulture.

Where asymmetrical carbon atoms are present in the compounds of formula I, these compounds are in optically active form. The invention relates to the pure isomers, such as enantiomers and diastereomers, as well as to all possible mixtures of isomers, e.g. mixtures of diastereomers, racemates or mixture of racemates.

Preferred embodiments of compounds of formula I are those wherein
R₁ is CF₃, CF₂H or CFH₂; or
R₁ is CF₃; or
R₂ is hydrogen or fluoro; or
R₂ is hydrogen; or
R₂ is fluoro; or
R₃ is hydrogen or fluoro; or
R₃ is hydrogen; or
R₃ is fluoro; or
R₄ is hydrogen, chloro, methyl, CF₃ or OCF₃; or
R₄ is hydrogen or methyl; or
R₄ is hydrogen, or
R₂, R₃ and R₄ are all hydrogen.

Within the group of compounds of formula I those compounds are preferred wherein R₁ is CF₃, CF₂H or CFH₂;
R₂ is hydrogen or fluoro;
R₃ is hydrogen or fluoro; and
R₄ is hydrogen, chloro, methyl, CF₃ or OCF₃ (subgroup A).

Within the subgroup A are those compounds preferred wherein
R₁ is CF₃, CF₂H or CFH₂;
R₂ is hydrogen;
R₃ is hydrogen or fluoro; and
R₄ is hydrogen, chloro, methyl, CF₃ or OCF₃ (subgroup A1).

Another group of compounds of formula I within the subgroup A are those wherein
R₁ is CF₃, CF₂H or CFH₂;
R₂ is fluoro;
R₃ is hydrogen or fluoro; and
R₄ is hydrogen, chloro, methyl, CF₃ or OCF₃ (subgroup A2).

Among these subgroups, those compounds are preferred wherein R₂, R₃ and R₄ are all hydrogen.

Preferred individual compounds are:
1-methyl-4-trifluoromethyl-1 H-pyrrole-3-carboxylic acid (4'-bromobiphenyl-2-yl) amide;
1-methyl-4-difluoromethyl-1 H-pyrrole-3-carboxylic acid (4'-bromobiphenyl-2-yl) amide.

The compounds according to formula I may be prepared according to the following reaction in schemes. (Ac designates an acetyl group).

### A) Synthesis of the pyrrole carboxylic acids :

### Route 1 (Tosmic-route)

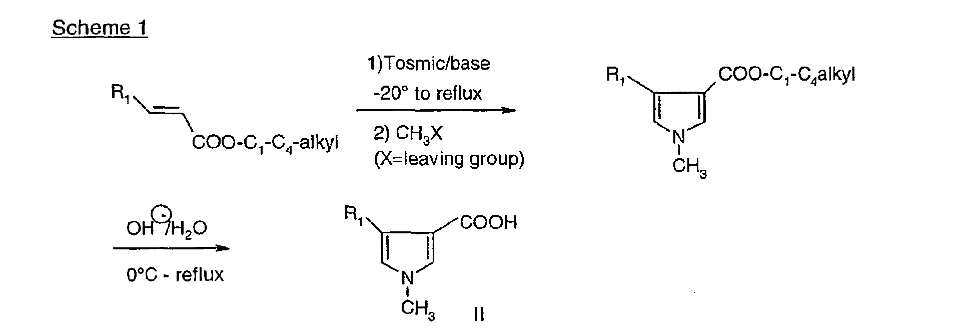

### Route 2 (Trifluoroacetoacetic acid-route, analogous to JP-07157466)

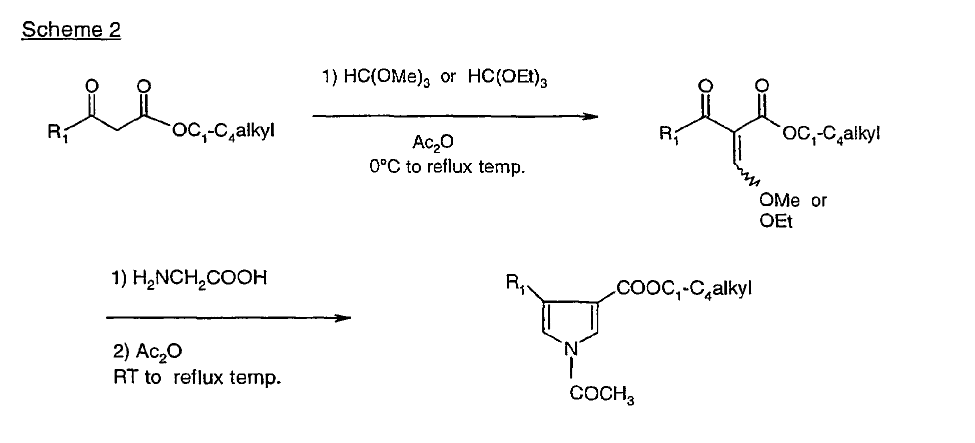

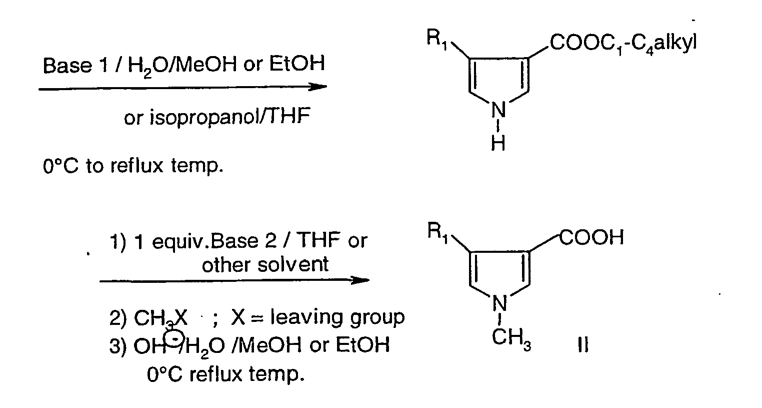

Base 1 = NaHCO₃, Na₂CO₃, KHCO₃, K₂CO₃, CaCO₃ and other bases
Base 2 = NaOH, KOH, NaH, KH, n-BuLi and others

The synthesis of the pyrrole carboxylic acids of formula II wherein R₂ = H is described in WO-00/09482.

The synthesis of the pyrrole carboxylic acids of formula II wherein R₂ is fluoro may be conducted according to the Schemes 2A or 2B. 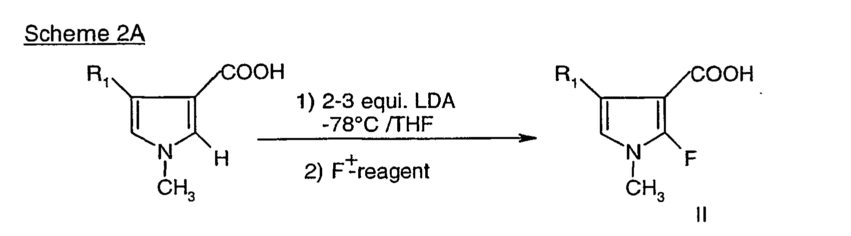
- F⁺-reagents: = N-fluoro-bis(phenylsulfonyl)amine, N-fluoro-N-methyl-toluene-4-sulfonamide, 2-fluoro-3,3-dimethyl-2,3-dihydro-1,2-benzisothiazole-1,1-dioxide, 1-fluoro-sym.-collidiniumtetrafluoroborate
LDA = lithiumdiisopropylamide

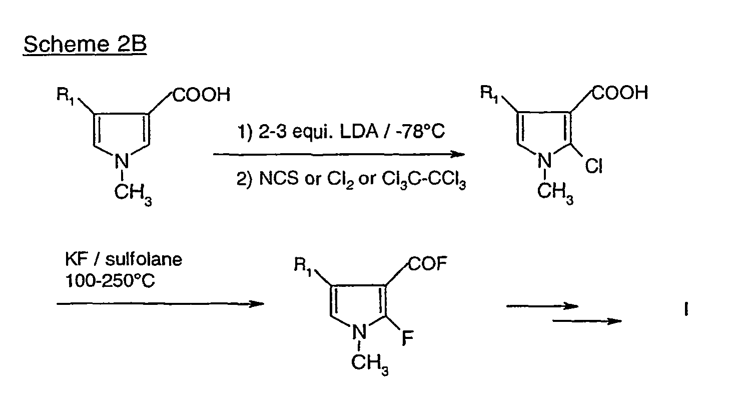

### B) synthesis of the amine III

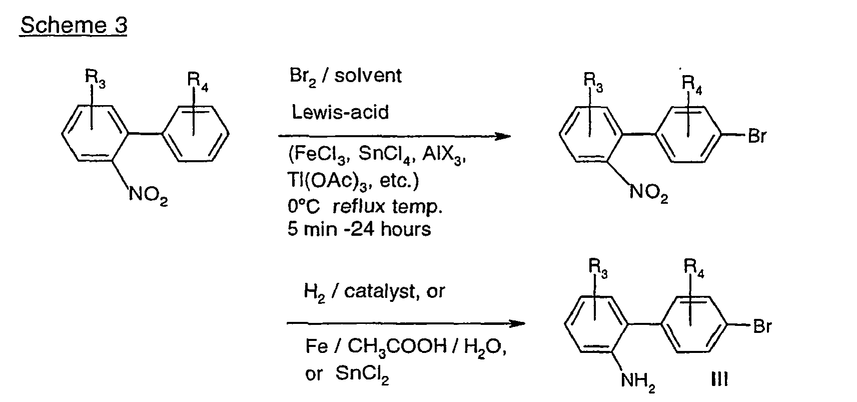

(AIX₃ is preferably AlCl₃; solvent: water, DMF, THF, CH₂Cl₂, CHCl₃, ClCH₂CH₂Cl, etc.)

### C) Synthesis of the amides

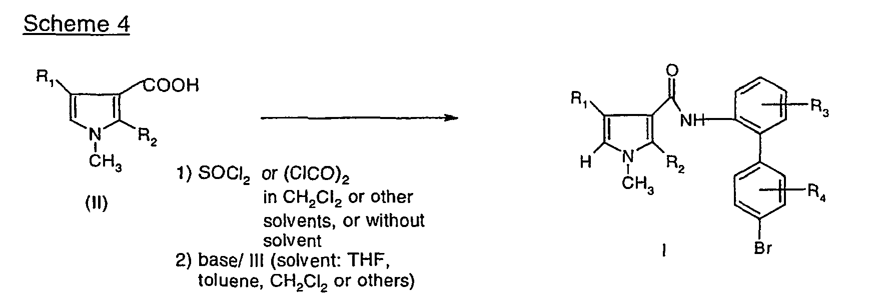

Base = N(C₂H₅)₃, Hünig-base, Na₂CO₃, K₂CO₃ and others

The pyrrole carboxylic acid II reacts with an activating agent such as thionyl chloride, phosphorous pentachloride or oxalic acid (or oxalyl) chloride in the presence of a solvent at a temperature between 0°C and reflux temperature and a reaction time of 30 minutes to 24 hours to give the corresponding acid chloride. Representative solvents are toluene, benzene, xylene, hexane, cyclohexane chloroform or methylenechloride. The obtained acyl chloride are normally not isolated. The new carboxamides of formula I are preferably obtained by reacting the activated pyrrolecarboxylic acid with an aromatic amine of formula III in the presence of a solvent like toluene, benzene, xylene, hexane, cyclohexane chloroform or methylenechloride and in the presence of an acid binding agent like triethylamine, Hünig base, sodium carbonate, potassium carbonate or sodium hydrogen carbonate at a temperature between 0°C and reflux temperature. Preferably the entire reaction sequence of scheme 4 is conducted as a single-vessel reaction.

The carboxylic acid fluoride intermediates of formula II wherein R₁ is CHF₂ may be obtained according to the following reaction route: 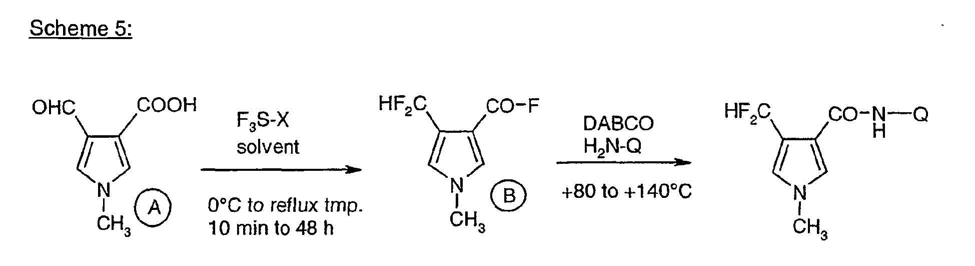 wherein Q is radical 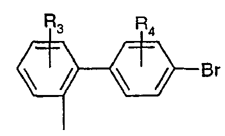 as defined as part of formula I and X is F, -N(CH₃)₂, -N(C₂H₅)₂ , -N(CH₂CH₂OCH₃)₂ or 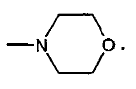 The reagents F₃S-X are known, e.g. from J.Org.Chem, 1999, (64), 7048.
The last reaction step of Scheme 5 comprisis a coupling (amidation) reaction which is advantageously conducted in the presense of 1 to 2 equivalents of a sterically hindered base like DABCO (1,4-diazabicyclo[2.2.2]octane) in a few drops of acetonitrile as a contact medium, by heating the reaction mixture for 2 to 3 hours to a temperature of 80°C to 140°C until the reaction has taken place. After coling routine work-up procedure yields the final product of formula I wherein R₁ is CHF₂.

Compound A may be obtained as follows. 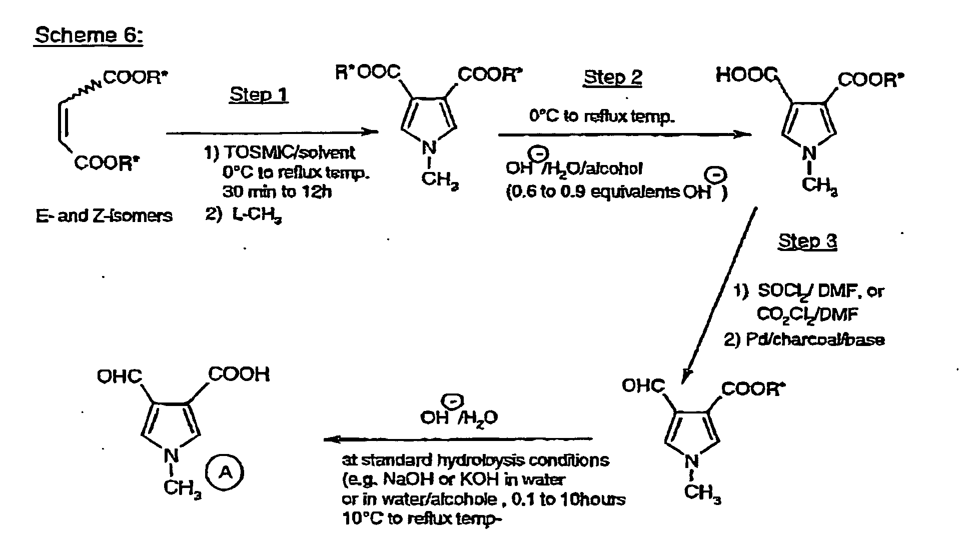 wherein R* is C₁-C₆alkyl, or Si(C₁-C₆alkyl)₃, and L is a leaving group like a halogen atom, -O-Tos , etc.. Reaction Step 3 is a modified version of a Rosenmund reaction, which is not yet known in the art, and thus represents another feature of this invention. Specifics are reactant combinations SOCl₂/DMF or oxalic acid dichloride/DMF, DMF in catalytic amount, optional inert solvent, in the first step and in the second step: reducing conditions like Pd on carbon at a temperature between 0°C and +10°C, preferably between 0°C and +5°C, and advantageously in the presence of tertiary amine, like e.g. a Hünig-base.

Alternatively, the 3,4-diester-1-methylpyrrole of Scheme 6 may be obtained according to R.K.Huisgen, US 3,285,931) as outlined in scheme 7.

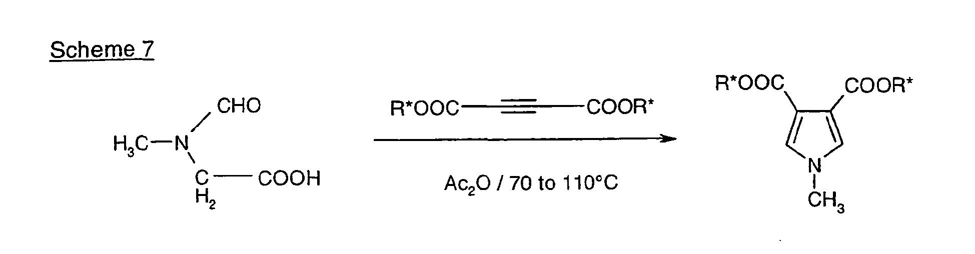

Surprisingly, it has now been found that the novel compounds of formula I have, for practical purposes, a very advantageous spectrum of activities for protecting plants against diseases that are caused by fungi as well as by bacteria and viruses.

The compounds of formula I can be used in the agricultural sector and related fields of use as active ingredients for controlling plant pests. The novel compounds are distinguished by excellent activity at low rates of application, by being well tolerated by plants and by being environmentally safe. They have very useful curative, preventive and systemic properties and are used for protecting numerous cultivated plants. The compounds of formula I can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula I as dressing agents for the treatment of plant propagation material, in particular of seeds (fruit, tubers, grains) and plant cuttings (e.g. rice), for the protection against fungal infections as well as against phytopathogenic fungi occurring in the soil.

The compounds I are, for example, effective against the phytopathogenic fungi of the following classes: Fungi imperfecti (e.g. Botrytis, Pyricularia, Helminthosporium, Fusarium, Septoria, Cercospora and Alternaria) and Basidiomycetes (e.g. Rhizoctonia, Hemileia, Puccinia). Additionally, they are also effective against the Ascomycetes classes (e.g. Venturia and Erysiphe, Podosphaera, Monilinia, Uncinula) and of the Oomycetes classes (e.g. Phytophthora, Pythium, Plasmopara). Outstanding activity has been observed against powdery mildew (Erysiphe spp.). Furthermore, the novel compounds of formula I are effective against phytopathogenic bacteria and viruses (e.g. against Xanthomonas spp, Pseudomonas spp, Erwinia amylovora as well as against the tobacco mosaic virus).

Within the scope of present invention, target crops to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as ornamentals.

The compounds of formula I are used in unmodified form or, preferably, together with the adjuvants conventionally employed in the art of formulation. To this end they are conveniently formulated in known manner to emulsifiable concentrates, coatable pastes, directly sprayable or dilutable solutions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula I are normally used in the form of compositions and can be applied to the crop area or plant to be treated, simultaneously or in succession with further compounds. These further compounds can be e.g. fertilizers or micronutrient donors or other preparations which influence the growth of plants. They can also be selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula I can be mixed with other fungicides, resulting in some cases in unexpected synergistic activities. Mixing components which are particularly preferred are azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, pyrifenox, prochloraz, propiconazole, simeconazole, tebuconazole, tetraconazole, triadimefon, triadimenol, triflumizole, triticonazole; pyrimidinyl carbinole, such as ancymidol, fenarimol, nuarimol; 2-amino-pyrimidines, such as bupirimate, dimethirimol, ethirimol; morpholines, such as dodemorph, fenpropidine, fenpropimorph, spiroxamine, tridemorph; anilinopyrimidines, such as cyprodinil, mepanipyrim, pyrimethanil; pyrroles, such as fenpiclonil, fludioxonil; phenylamides, such as benalaxyl, furalaxyl, metalaxyl, R-metalaxyl, ofurace, oxadixyl; benzimidazoles, such as benomyl, carbendazim, debacarb, fuberidazole, thiabendazole; dicarboximides, such as chlozolinate, dichlozoline, iprodione, myclozoline, procymidone, vinclozoline; carboxamides, such as carboxin, fenfuram, flutolanil, mepronil, oxycarboxin, thifluzamide; guanidines, such as guazatine, dodine, iminoctadine; strobilurines, such as azoxystrobin, kresoxim-methyl, metominostrobin, SSF-129, trifloxystrobin, picoxystrobin, BAS 500F (proposed name pyraclostrobin), BAS 520; dithiocarbamates, such as ferbam, mancozeb, maneb, metiram, propineb, thiram, zineb, ziram; N-halomethylthiotetrahydrophthalimides, such as captafol, captan, dichlofluanid, fluoromides, folpet, tolyfluanid; Cu-compounds, such as Bordeaux mixture, copper hydroxide, copper oxychloride, copper sulfate, cuprous oxide, mancopper, oxine-copper; nitrophenol-derivatives, such as dinocap, nitrothal-isopropyl; organo-p-derivatives, such as edifenphos, iprobenphos, isoprothiolane, phosdiphen, pyrazophos, tolclofosmethyl; various others, such as acibenzolar-S-methyl, anilazine, benthiavalicarb, blasticidin-S, chinomethionate, chloroneb, chlorothalonil, cyflufenamid, cymoxanil, dichlone, diclomezine, dicloran, diethofencarb, dimethomorph, SYP-LI90 (proposed name: flumorph), dithianon, ethaboxam, etridiazole, famoxadone, fenamidone, fenoxanil, fentin, ferimzone, fluazinam, flusulfamide, fenhexamid, fosetyl-aluminium, hymexazol, iprovalicarb, IKF-916 (cyazofamid), kasugamycin, methasulfocarb, metrafenone, nicobifen, pencycuron, phthalide, polyoxins, probenazole, propamocarb, pyroquilon, quinoxyfen, quintozene, sulfur, triazoxide, tricyclazole, triforine, validamycin, zoxamide (RH7281).

A preferred method of applying a compound of formula I, or an agrochemical composition which contains at least one of said compounds, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

The formulation, i.e. the compositions containing the compound of formula I and, if desired, a solid or liquid adjuvant, are prepared in known manner, typically by intimately mixing and/or grinding the compound with extenders, e.g. solvents, solid carriers and, optionally, surface active compounds (surfactants).

The agrochemical formulations will usually contain from 0.1 to 99 % by weight, preferably from 0.1 to 95 % by weight, of the compound of formula I, 99.9 to 1 % by weight, preferably 99.8 to 5 % by weight, of a solid or liquid adjuvant, and from 0 to 25 % by weight, preferably from 0.1 to 25 % by weight, of a surfactant.

Advantageous rates of application are normally from 5 g to 2 kg of active ingredient (a.i.) per hectare (ha), preferably from 10 g to 1 kg a.i./ha, most preferably from 20 g to 600 g a.i./ha. When used as seed drenching agent, convenient dosages are from 10 mg to 1 g of active substance per kg of seeds.

Whereas it is preferred to formulate commercial products as concentrates, the end user will normally use dilute formulations.

The following non-limiting Examples illustrate the above-described invention in more detail. Temperatures are given in degrees Celsius. The following abbreviations are used: m.p.= melting point; b.p.= boiling point. "NMR" means nuclear magnetic resonance spectrum. MS stands for mass spectrum. "%" is percent by weight, unless corresponding concentrations are indicated in other units.

### Example 1

### 1-Methyl-4-trifluoromethyl-1H-pyrrole-3-carboxylic acid (4'-bromobiphenyl-2-yl) amide

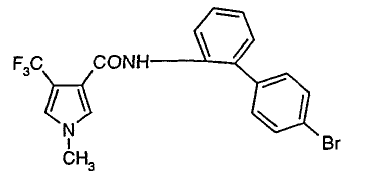

A solution of 1-methyl-4-trifluoromethyl-1H-pyrrole-3-carboxylic acid (0.42 g, 2.2 mmol) and oxalyl chloride (0.30 g, 2.4 mmol) in methylene chloride (20 ml) is stirred for 3 hours at room temperature in the presence of a catalytic amount of DMF. Then the resulting acid chloride solution is slowly added to a solution of 2-(4'-bromophenyl)aniline (0.55 g, 2.2 mmol) and triethylamine (0.33 g, 3.3 mmol) in 15 ml of methylene chloride. The resulting mixture is then stirred for 16 hours at room temperature. After the addition of ethylacetate, the organic phase is washed twice with water. After drying the organic phase over Na₂SO₄, the solvent is removed in a water-jet-vacuum and the obtained crude product is finally purified by column chromatography (silica gel; eluant: ethylacetate/hexane=1:2). 0.52 g of 1-methyl-4-trifluoromethyl-1 H-pyrrole-3-carboxylic acid (4'-bromobiphenyl-2-yl) amide are obtained in the form of a yellow powder having a melting point of 163-164°C.

The following compounds of formula I are prepared in a similar way, using analogous methods.

**Table 1**

| | | | | | |
|---|---|---|---|---|---|
| 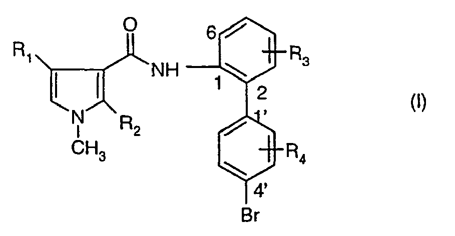 | | | | | |

| Compd. No. | R₁ | R₂ | R₃ | R₄ | phys.data, m.p. °C |
|---|---|---|---|---|---|
| 1.1 | CF₃ | H | H | H | 163-164 |
| 1.2 | CF₂H | H | H | H | 166-167 |
| 1.3 | CFH₂ | H | H | H | |
| 1.4 | CF₃ | F | H | H | resin, M⁺=411 |
| 1.5 | CF₂H | F | H | H | resin, M⁺=423 |
| 1.6 | CFH₂ | F | H | H | |
| 1.7 | CF₃ | H | 6-F | H | |
| 1.8 | CF₂H | H | 6-F | H | |
| 1.9 | CF₃ | F | 6-F | H | |
| 1.10 | CF₂H | F . | 6-F | H | |

### Reference Example 2: 4-Formyl-1-methyl-1H-pyrrole-carboxylic acid ethylester

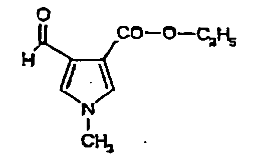

A solution of 21.6 g (0-11 mol) 1-methyt-1H-pyrrazole-3,4-dicarboxylic acid monoethylester and 14.9 g (0.117 mol) oxalyl chloride and 150 ml methylene chloride is stirred for 3 hours at room temperature in the presence of a catalytic amount of absolute DMF. After 3 hours the solvent is remorvd in a water jet vacuum and the crude acid chloride (21.5 g) is dissolved in 400 ml of dry tetrahydrofurane. After addition of 14.2 g (0.11 mol) of N,N-diisopropylethylamine (Hünig-base) the mixture is hydrogenated with hydrogen in the presence of 6.0 g 10% Pd/C at 0°-5°C for 5 ½ hours. Then the catalyst is filtered off and the solvent removed in a water jet vacuum. The raw material is purified by flash-chromatography over silica gel (eluant: t-butylmethylether/hexane 1:5). Yield: 16 g 4-formyl-1-methyl-1 H-pyrrole carboxylic acid ethylester in the form of a yellow powder, m.p.: 75°-76°C.

### Reference Exemple 3: 4-Formyl-1-methyl-1H-pyrrole-carboxylic acid

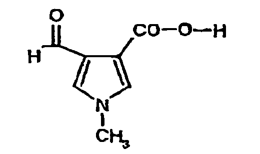

To a solution of 4.6 g (0.0255 mol) 4-formyl-7-methyl-1H-pyrrole-carboxylic acid ethylester and 90 ml of ethanol is added a solution of 2.1 g (0.031 mol) potassium hydroxide of 85% and 20 ml of water producing a slightly exothermic reaction. The resulting mixture is stirred for 2 hours at +80°C and then the solvent mixture EtOH/H₂O is distilled off in vacuo. The resulting oil is dissolved in 100 ml of H₂O and washed twice with ethylacetate. Then 20 ml of 2N hydrogen chloride solution is added slowly of the water phase in the cold. The precipitated solid is filtered off and washed with water. After drying of the precipitate in a vacuum oven the pure acid is obtained. Yield: 3.6 g of 4-formyl-1-methyl-1H-pyrrolecarboxylic acid in the form of a slightly yellow powder; m.p.: 178°-180°C.

### Reference Example 4

### 4-Difluoromethyl-1-methyl-1H-pyrrole-3-carbonyl fluoride

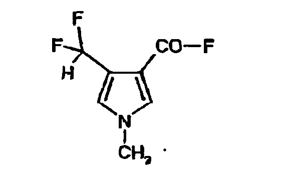

To a cooled solution of 2.6 g (0.017 mol) 4-formyl-methyl-1H-pyrrole-carboxylic acid and 70 ml methylene chloride is added a solution of 11.0 g (0.068 mol) diethylaminosulfurtrifluoride (DAST) and 10 ml methylenechloride in such a manner that the temperature remains constant at 0° to +2°C. Then the mixture is stirred for 30 minutes at 0°C and 16 hours at room temperature. The reaction mixture is taken up in ethylacetate and washed twice with ice water and brine. After drying the solvent is removed in a water jet vacuum and the residue purified by flash chromatography over silica gel (eluant hexane/ethylacetate. 2:1). Yield: 1.95 g 4-difluoromethyl-1-methyl-1H-pyrrole-3-carbonyl fluoride in the form of a brownish solid; m.p.: 53°-54°C.

In a similar manner the intermediates of the general formula B of table 2 may be obtained.

**Table 2**

| | | |
|---|---|---|
| 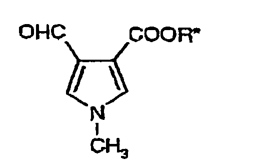 | | |

| Compound. No. | R* | phys. Data (m.p. [°C]) |
|---|---|---|
| 2.1 | CH₃ | |
| 2.2 | C₂H₅ | 76-77 |
| 2.3 | C₂H₇-n | |
| 2.4 | C₄H₉-n | |
| 2.5 | C₆H₁₃-n | |
| 2.6 | Si(CH₃)₃ | |

### Formulation Examples for compounds of formula I

Working procedures for preparing formulations of the compounds of formula I such as Emulsifiable concentrates, Solutions, Granulates, Dusts and Wettable powders are described in WO 97/33890.

### Biological Examples: Fungicidal actions

### Example B-1: Action against Puccinia recondita /wheat (Brownrust on wheat)

1 week old wheat plants cv. Arina are treated with the formulated test compound (0.02% active ingredient) in a spray chamber. One day after application wheat plants are inoculated by spraying a spore suspension (1 x 10⁵ uredospores/ml) on the test plants. After an incubation period of 2 days at 20° C and 95% r. h. plants are kept in a greenhouse for 8 days at 20° C and 60% r.h. The disease incidence is assessed 10 days after inoculation.

Compounds of Table 1 show good activity in this test (< 20% infestation). Infestation is prevented virtually completely (0-5% infestation) with compounds 1.1, 1.2, 1.4 and 1.5.

### Example B-2: Action against Podosphaera leucotricha / apple (Powdery mildew on apple)

5 week old apple seedlings cv. McIntosh are treated with the formulated test compound (0.002% active ingredient) in a spray chamber. One day after application apple plants are inoculated by shaking plants infected with apple powdery mildew above the test plants. After an incubation period of 12 days at 22° C and 60% r. h. under a light regime of 14/10 h (light/dark) the disease incidence is assessed.

Compounds of Table 1 show good activity in this test. The compounds 1.1, 1.2, 1.4 and 1.5 exhibit strong efficacy (< 20% infestation).

### Example B-3: Action against Venturia inaequalis l apple (Scab on apple)

4 week old apple seedlings cv. McIntosh are treated with the formulated test compound (0.02% active ingredient) in a spray chamber. One day after application apple plants are inoculated by spraying a spore suspension (4 x 10⁵ conidia/ml) on the test plants. After an incubation period of 4 days at 21° C and 95% r. h. the plants are placed for 4 days at 21° C and 60% r. h. in a greenhouse. After another 4 day incubation period at 21° C and 95% r. h. the disease incidence is assessed.
Compounds of Table 1 show good activity in this test. The compounds 1.1, 1.2, 1.4 and 1.5 exhibit strong efficacy (< 20% infestation).

### Example B-4: Action against Erysiphe graminis / barley (Powdery mildew on barley)

1 week old barley plants cv. Express are treated with the formulated test compound (0.02% active ingredient) in a spray chamber. One day after application barley plants are inoculated by shaking powdery mildew infected plants above the test plants. After an incubation period of 6 days at 20°C / 18°C (day/night) and 60% r. h. in a greenhouse the disease incidence is assessed.
Compounds of Table 1 show good activity in this test. The compounds 1.1, 1.2, 1.4 and 1.5 exhibit strong efficacy (< 20% infestation).

### Example B-5: Action against Botrytis cinerea / apple (Botrytis on apple fruits)

In an apple fruit cv. Golden Delicious 3 holes are drilled and each filled with 30 µl droplets of the formulated test compound (0.002% active ingredient). Two hours after application 50 µl of a spore suspension of *B*. *cinerea* (4 x 10⁵ conidia/ml) are pipetted on the application sites. After an incubation period of 7 days at 22° C in a growth chamber the disease incidence is assessed.
Compounds of Table 1 show good activity in this test. The compounds 1.1, 1.2, 1.4 and 1.5 exhibit very strong efficacy (< 10% infestation).

### Example B-6: Action against Botrytis cinerea / grape (Botrytis on grapes)

5 week old grape seedlings cv. Gutedel are treated with the formulated test compound (0.002% active ingredient) in a spray chamber. Two days after application grape plants are inoculated by spraying a spore suspension (1 x 10⁶ conidia/ml) on the test plants. After an incubation period of 4 days at 21° C and 95% r. h. in a greenhouse the disease incidence is assessed.
Compounds of Table 1 show good activity in this test. The compounds 1.1, 1.2, 1.4 and 1.5 exhibit very strong efficacy (< 10% infestation).

### Example B-7: Action against Botrytis cinerea / tomato (Botrytis on tomatoes)

4 week old tomato plants cv. Roter Gnom are treated with the formulated test compound (0.002% active ingredient) in a spray chamber. Two days after application tomato plants are inoculated by spraying a spore suspension (1 x 10⁵ conidia/ml) on the test plants. After an incubation period of 4 days at 20° C and 95% r. h. in a growth chamber the disease incidence is assessed.
Compounds of Table 1 show good activity in this test. The compounds 1.1, 1.2, 1.4 and 1.5 exhibit very strong efficacy (< 10% infestation).

### Example B-8: Action against Pyrenophora teres / barley (Net blotch on barley)

1 week old barley plants cv. Express are treated with the formulated test compound (0.002% active ingredient) in a spray chamber. Two days after application barley plants are inoculated by spraying a spore suspension (3 x 10⁴ conidia/ml) on the test plants. After an incubation period of 2 days at 20° C and 95% r. h. plants are kept for 2 days at 20° C and 60% r.h. in a greenhouse. The disease incidence is assessed 4 days after inoculation. Compounds of Table 1 show good activity in this test. The compounds 1.1, 1.2, 1.4 and 1.5 exhibit strong efficacy (< 20% infestation).

### Example B-9: Action against Septoria nodorum /wheat (Septoria leaf spot on wheat)

1 week old wheat plants cv. Arina are treated with the formulated test compound (0.02% active ingredient) in a spray chamber. One day after application wheat plants are inoculated by spraying a spore suspension (5 x 10⁵ conidia/ml) on the test plants. After an incubation period of 1 day at 20° C and 95% r. h. plants are kept for 10 days at 20° C and 60% r.h. in a greenhouse. The disease incidence is assessed 11 days after inoculation. Compounds of Table 1 show good activity in this test. The compounds 1.1, 1.2, 1.4 and 1.5 exhibit strong efficacy (< 20% infestation).

## Claims

1. A pyrrolecarboxamide of formula I 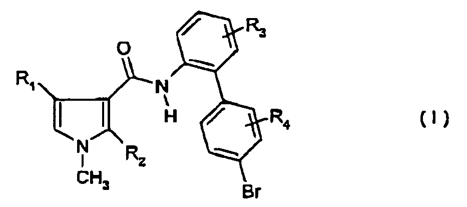 wherein
R₁ is CF₃, CF₂H or CFH₂;
R₂ is hydrogen or fluoro;
R₃ is hydrogen or fluoro; and
R₄ is hydrogen, fluoro, chloro, bromo, methyl, CF₃, OCF₃ or SCF₃.

2. A compound of formula I according to claim 1, wherein
R₄ is hydrogen, chloro, methyl, CF₃ or OCF₃.

3. A compound of formula I according to claim 2, wherein
R₂ is hydrogen.

4. A compound of formula I according to claim 2, wherein
R₂ is fluoro.

5. A compound of formula I according to daim 1 selected from the group comprising 1-methyl-4-trifluoromethyl-1H-pyrrole-3-carboxylic acid (4'bromobiphenyl-2-yl) amide or 1-methyl-4-difluoromethyl-1H-pyrrole-3-carboxylic acid (4'bromobiphenyl-2-yl) amide.

6. A process for the preparation of compounds of formula I which comprises reacting the starting materials according to the scheme 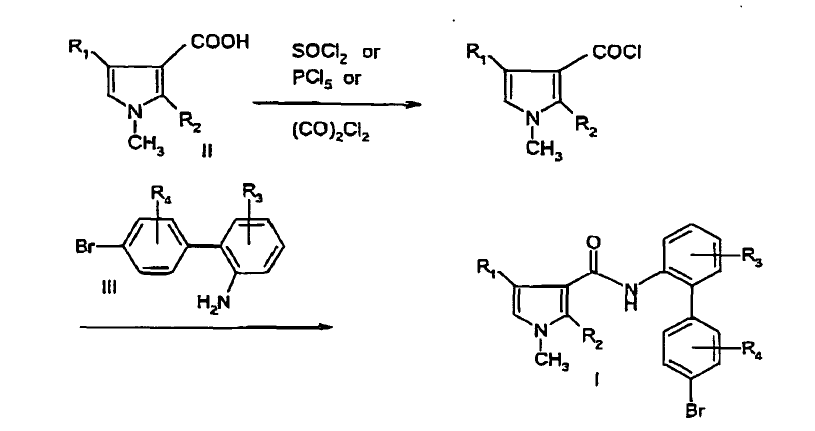 wherein R₁, R₂, R₃ and R₄ are as defined for formula I in claim 1.

7. A composition for controlling microorganisms and preventing attack and infestation of plants therewith, wherein the active ingredient is a compound as claimed in claim 1 together with a suitable carrier.

8. A method of controlling or preventing infestation of cultivated plants by phytopathogenic microorganisms by application of a compound of formula I as claimed in claim 1 to plants, to parts thereof or the locus thereof.

## Patentansprüche

1. Pyrrolcarboxamid der Formel 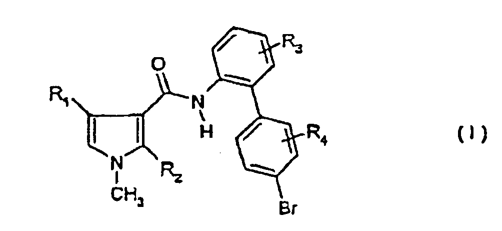 worin
R₁ CF₃, CF₂H oder CFH₂ ist;
R₂ Wasserstoff oder Fluor ist;
R₃ Wasserstoff oder Fluor ist und
R₄ Wasserstoff, Fluor, Chlor, Brom, Methyl, CF₃, OCF₃ oder SCF₃ ist.

2. Verbindung der Formel I nach Anspruch 1, worin R₄ Wasserstoff, Chlor, Methyl, CF₃ oder OCF₃ ist.

3. Verbindung der Formel I nach Anspruch 2, worin R₂ Wasserstoff ist.

4. Verbindung der Formel I nach Anspruch 2, worin R₂ Fluor ist.

5. Verbindung der Formel I nach Anspruch 1, ausgewählt aus der Gruppe, umfassend 1-Methyl-4-trifluormethyl-1H-pyrrol-3-carbonsäure-(4'-brombiphenyl-2-yl)amid oder 1-Methyl-4-difluormethyl-1H-pyrrol-3-carbonsäure-(4'-brombiphenyl-2-yl)amid.

6. Verfahren zur Herstellung von Verbindungen der Formel I, umfassend das Umsetzen der Ausgangsmaterialien gemäß dem Schema 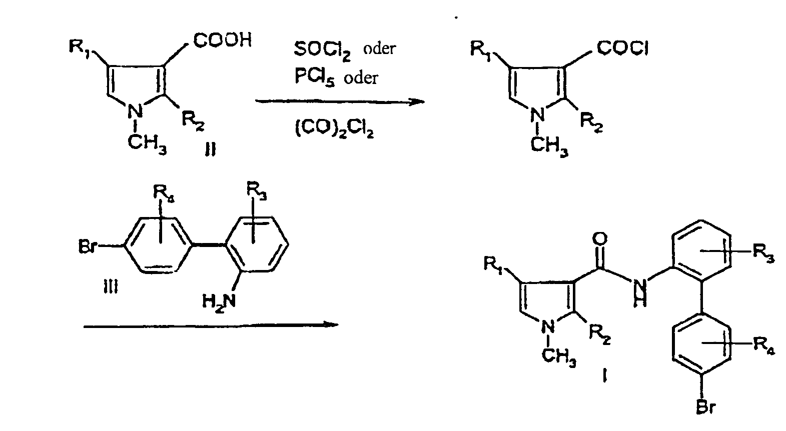 worin R₁, R₂, R₃ und R₄ wie für Formel I in Anspruch 1 definiert sind.

7. Zusammensetzung zur Bekämpfung von Mikroorganismen und Vorbeugung des Angriffs und des Befalls von Pflanzen damit, wobei der Wirkstoff eine Verbindung nach Anspruch 1 ist, zusammen mit einem geeigneten Träger.

8. Verfahren zur Bekämpfung oder Vorbeugung des Befalls von kultivierten Pflanzen durch phytopathogene Mikroorganismen durch Applikation einer Verbindung der Formel I nach Anspruch 1 auf Pflanzen, auf Teile davon oder deren Standort.

## Revendications

1. Pyrrolecarboxamide de formule I 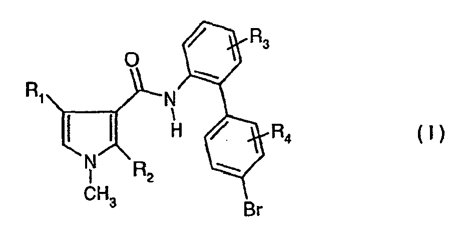 dans laquelle
R₁ est CF₃, CF₂H ou CFH₂ ;
R₂ est un atome d'hydrogène ou un atome de fluor ;
R₃ est un atome d'hydrogène ou un atome de fluor ; et
R₄ est un atome d'hydrogène, un atome de fluor, un atome de chlore, un atome de brome, un groupe méthyle, CF₃, OCF₃ ou SCF₃.

2. Composé de formule I selon la revendication 1, dans laquelle R₄ est un atome d'hydrogène, un atome de chlore, un groupe méthyle, CF₃ ou OCF₃.

3. Composé de formule I selon la revendication 2, dans laquelle R₂ est un atome d'hydrogène.

4. Composé de formule I selon la revendication 2, dans laquelle R₂ est un atome de fluor.

5. Composé de formule I selon la revendication 1 choisi dans le groupe comprenant
le (4'-bromobiphényl-2-yl)-amide de l'acide 1-méthyl-4-trifluorométhyl-1H-pyrrole-3-carboxylique ou
le (4'-bromobiphényl-2-yl)-amide de l'acide 1-méthyl-4-difluorométhyl-1H-pyrrole-3-carboxylique.

6. Procédé de préparation des composés de formule I qui comprend la mise en réaction des produits de départ selon le schéma 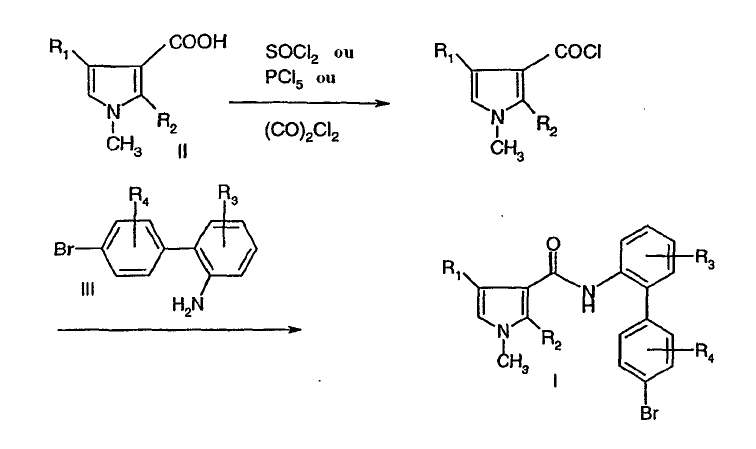 où R₁, R₂, R₃ et R₄ sont tels que définis pour la formule I selon la revendication 1.

7. Composition pour contrôler les microorganismes et empêcher l'attaque et l'invasion des plantes par ces microorganismes, dans laquelle l'ingrédient actif est un composé selon la revendication 1 avec un support approprié.

8. Procédé de contrôle et de prévention d'une invasion de plantes Cultivées par des microorganismes phytopathogènes par l'application d'un composé de formule I selon la revendication 1 aux plantes, à des parties de celles-ci ou au locus de celles-ci.
